# EUROPÄISCHE PATENTANMELDUNG

(11) **EP 0 564 681 A1**
(43) Veröffentlichungstag der Anmeldung: **13.10.1993**
(21) Anmeldenummer: 92106112.3
(22) Anmeldetag: 09.04.1992
(51) Int. Cl.: A61F 13/22

(54) **Tampon**

(71) Anmelder: SANPOINT AB, S-647000 Mariefred (SE)
(72) Erfinder: de Groot, Hans Feikes, S-645 94 Strängnäs (SE)
(74) Vertreter: COHAUSZ HASE DAWIDOWICZ & PARTNER

(57) **Zusammenfassung**

Die Erfindung betrifft einen Tampon für die Frauenhygiene mit einem gewickelten Kern (1) aus einem Faservlies und einem den Kern außen umgebenden Mantel (2) mit thermoplastischen Fasern, wobei der Mantel aus einem Faservlies besteht, das zusätzlich zu den thermoplastischen Fasern hydrophile Fasern aufweist.

## Beschreibung

Die Erfindung betrifft einen Tampon für die Frauenhygiene mit einem gewickelten Kern aus einem Faservlies und einem den Kern außen umgebenden Mantel mit thermoplastischen Fasern.

Es sind Mäntel (Umhüllungen) von Tampons bekannt, die vollsynthetisch sind und hydrophobische Eigenschaften besitzen. Um ein Lecken zu vermeiden, muß der Mantel immer großflächig mit dem absorbierenden Kern verbunden sein, so daß eine aufwendige Auflage erforderlich ist.

Aufgabe der Erfindung ist es, einen Tampon der eingangs genannten Art zu schaffen, der bei einfacher Herstellung, die Sicherheit bietet, daß der Mantel sich bei der Anwendung, d.h. bei Feuchtwerden sofort an den absorbierenden Kern anschmiegt und damit ein Durchlecken zwischen Mantel und Kern verhindert wird.

Diese Aufgabe wird erfindungsgemäß dadurch gelöst, daß der Mantel aus einem Faservlies besteht, das zusätzlich zu den thermoplastischen Fasern hydrophile Fasern aufweist, die bei Feuchtigkeit sich erweichen.

Ein solcher Tampon ist in der Herstellung wenig aufwendig und bietet eine hohe Sicherheit in der Anwendung. Beim Feuchtwerden schmiegt sich der Mantel, bedingt durch das Erweichen des zellulosischen Faseranteils an den Kern an, so daß ein optimales Verhalten erreicht wird. Durch den hydrophilen Charakter des Mantels wird Flüssigkeit nicht nur sehr schnell absorbiert und durch die enge Auflage durch den Kern schnell weitergegeben, sondern es ist auch eine vollständige und großflächige Verschweißung des Mantels mit dem Kern nicht erforderlich. Vielmehr reicht es aus, wenn der Mantel mit dem Kern durch eine einzige Verbindungsstelle verbunden ist, die linien- oder streifenförmig sein kann und vorzugsweise quer zur Längsrichtung des gewickelten Kernbandes liegt.

Hierbei ist es von besonderer Bedeutung, wenn die Verbindungsstelle durch eine Wickellage außen überdeckt ist. Auch wird vorgeschlagen, daß die Breite des Mantels gleich der Länge des Tampons ist. Hierdurch wird das Risiko des Faserablösens bei Gebrauch wesentlich verringert.

Besonders vorteilhaft ist es, wenn die hydrophilen Fasern einen Anteil von 10 bis 80 Prozent insbesondere ca. 50 Prozent haben. Hierbei können die hydrophilen Fasern vorteilhaft aus Zellstoff, insbesondere Viskose Faser oder Baumwolle bestehen.

Ein Ausführungsbeispiel ist in den Zeichnungen dargestellt und wird im folgenden näher beschrieben.

Es zeigen:

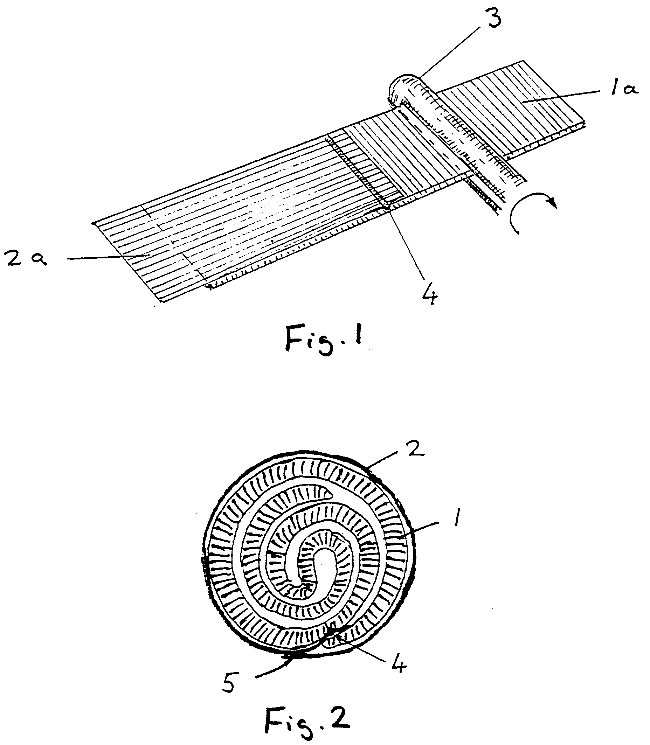

- Figur 1:: Eine perspektivische Darstellung des Kernbandes mit darauf befestigtem Mantelband und darübergeschobener Wickelgabel und
- Figur 2:: einen Querschnitt durch den Tampon im gewickelten Zustand vor dem Zusammenpressen.

Der Tampon weist einen inneren, etwa zylindrischen Kern 1 und einen diesen außen umgebenden Mantel 2 auf. Der Kern 1 ist aus einem Watteband 1a durch eine Wickelgabel 3 gewickelt und besteht aus absorptionsfähigen Natur- und/oder Kunststoffasern.

Der Mantel 2 besteht aus einem Mantelband 2a, das um den Kern 1 gewickelt ist und aus einem Faservlies besteht. Dieses Faservlies besteht aus einer Mischung von thermoplastischen und hydrophilen Fasern, wobei der Anteil hydrophiler Faser, Viskose Faser oder Baumwolle 10-80 Prozent, insbesondere ca. 50 Prozent beträgt.

Als thermoplastische Fasern sind alle Fasertypen geeignet, die sich unter Wärme und Druck verschweißen lassen. Insbesondere Polyten oder Polypropenfaser homogen oder heterophil, oder anderen Fasern mit einem hochschmelzendem Kern und niedrigschmelzendem Mantel, z.B. Polyester-Kern und Polyten-Mantel (sog. Heterophil- oder Bikomponent-Faser).

Die hydrophilen Fasern des Mantels 2 bestehen aus Zellstoff, insbesondere aus Viskose Faser oder Baumwolle.

Das Mantelband 2a ist auf dem Kernband 1a durch eine Schweißstelle 4 befestigt, die als Schweißnaht quer zur Längsrichtung der Bänder 1a, 2a liegt, wobei das Mantelband 2a das Kernband 1a weit überdeckt und nur soweit am äußeren Ende das Watteband überragt, daß das Mantelband 2a im gewickelten Zustand auf sich selbst befestigbar ist, insbesondere durch Schweißpunkte oder eine Schweißlinie.

Die Schweißstelle 4 liegt soweit innerhalb des Kernwickels, daß sie vom äußeren Ende des Kernbandes 1a überdeckt ist.

## Patentansprüche

1. Tampon für die Frauenhygiene mit einem gewickelten Kern (1) aus einem Faservlies und einem den Kern (1) außen umgebenden Mantel (2) mit thermoplastischen Fasern, **dadurch gekennzeichnet,** daß der Mantel (2) aus einem Faservlies besteht, das zusätzlich zu den thermoplastischen Fasern hydrophile Fasern aufweist.

2. Tampon nach Anspruch 1, **dadurch gekennzeichnet,** daß die hydrophilen Fasern einen Anteil von 10 bis 80 Prozent insbesondere ca. 50 Prozent haben.

3. Tampon nach Anspruch 1 oder 2, **dadurch gekennzeichnet,** daß die hydrophilen Fasern aus Zellstoff, insbesondere Viskose Faser oder Baumwolle bestehen.

4. Tampon nach einem der vorherigen Ansprüche, **dadurch gekennzeichnet,** daß der Mantel (2) mit dem Kern (1) durch eine einzige Verbindungsstelle (4) verbunden ist.

5. Tampon nach Anspruch 4, **dadurch gekennzeichnet,** daß die Verbindungsstelle (4) linien- oder streifenförmig ist und quer zur Längsrichtung des gewickelten Kernbandes (1a) liegt.

6. Tampon nach einem der vorhergehenden Ansprüche 4 oder 5, **dadurch gekennzeichnet,** daß die Verbindungsstelle (4) durch eine Wickellage außen überdeckt ist.

7. Tampon nach einem der vorherigen Ansprüche, **dadurch gekennzeichnet,** daß die Breite des Mantels (2) gleich der Länge des Tampons ist.
